# EUROPÄISCHE PATENTSCHRIFT

(11) **EP 2 162 419 B1**
(45) Veröffentlichungstag und Bekanntmachung des Hinweises auf die Patenterteilung: **23.07.2014**
(21) Anmeldenummer: 08750179.7
(22) Anmeldetag: 08.05.2008
(51) Int. Cl.: C07C 67/03, C07C 69/54

(54) **VERFAHREN ZUR HERSTELLUNG VON ETHYLENGLYCOLDIMETHACRYLAT**
METHOD FOR PRODUCING ETHYLENE GLYCOL DIMETHACRYLATE
PROCÉDÉ DE PRODUCTION DE DIMÉTHACRYLATE D'ÉTHYLÈNEGLYCOL

(30) Priorität: 05.07.2007 DE 102007031473
(43) Veröffentlichungstag der Anmeldung: 17.03.2010
(73) Patentinhaber: Evonik Röhm GmbH, 64293 Darmstadt (DE)
(72) Erfinder: KNEBEL, Joachim, 64665 Alsbach-hähnlein (DE); SCHÜTZ, Thorben, 64342 Seeheim-Jugenheim (DE); PROTZMANN, Guido, 64625 Bensheim (DE); TRAUTHWEIN, Harald, 68642 Bürstadt (DE); LAUSTER, Günther, 67547 Worms (DE); KEHR, Thomas, 64367 Muehltal (DE); KÖLBL, Gerhard, 64579 Gernsheim (DE); WESTHÄUSER, Günter, 67583 Guntersblum (DE)
(86) Internationale Anmeldenummer: PCT/EP2008/055668
(87) Internationale Veröffentlichungsnummer: WO 2009/003745

(56) Entgegenhaltungen:
- EP-A- 0 534 666
- EP-A- 1 231 202
- WO-A-2007/031384
- US-A- 4 672 105

## Beschreibung

Die vorliegende Erfindung betrifft Verfahren zur Herstellung von Ethylenglycoldi methacrylat.

Ethylenglycoldimethacrylat ist ein weithin bekanntes und verwendetes Monomer, welches insbesondere zur Vernetzung eingesetzt wird. Dementsprechend sind vielfältige Methoden bekannt, um diese Verbindungen zu erhalten. Hierzu gehören insbesondere Umesterungsreaktionen, bei denen Methylmethacrylat mit Ethylenglycol umgesetzt wird. Zur Verbesserung der Ausbeute und der Selektivität der Reaktion können unterschiedliche Katalysatoren eingesetzt werden.

Beispielsweise beschreibt die Druckschrift DE 28 05 702 die Herstellung von Estern ungesättigter Carbonsäuren. Zur Katalyse der beschriebenen Reaktionen können insbesondere Verbindungen eingesetzt werden, die Zirkonium und/oder Calcium enthalten. Zu den besonders geeigneten Katalysatoren gehört insbesondere Zirkoniumacetylacetonat. Die Umsetzungen führen zu hohen Ausbeuten von ca. 97%, bezogen auf den eingesetzten Alkohol. Nachteilig ist jedoch, dass der Katalysator nur sehr schwer aus der Reaktionsmischung abgetrennt werden kann und speziell mit Ethylenglycol als Reaktionspartner keine Wirkung zeigt.

Ein Verfahren zur Abtrennung dieses Katalysators wird zwar in DE 199 40 622 dargelegt, jedoch ist das Verfahren in der Durchführung relativ teuer.

Des Weiteren können Säuren oder Basen eingesetzt werden, um die Umesterung zu katalysieren. Derartige Reaktionen werden beispielsweise in CN 1355161, DE 34 23 443 oder EP-A-0 534 666 dargelegt. Bei Einsatz dieser Katalysatoren muss allerdings mit Nebenreaktionen gerechnet werden, wie beispielsweise der Michael-Addition, die sowohl die Reinheit des gewünschten Ethylenglycoldimethacrylats als auch die Ausbeute schmälert. Zu den basischen Katalysatoren gehört insbesondere Lithiumamid, wie dies beispielhaft in den Druckschriften DE 34 23 443, CA 795814 und US 6,194,530 dargelegt wird. Die Kombination von Lithiumamid mit weiteren Katalysatoren wird jedoch nicht beschrieben.

EP-A-1 231 202 offenbart die Umesterung von (Meth)acrylsäureestern in Gegenwart von Lithiumchlorid und Natriumcyanat als Katalysatorsystem.

In Anbetracht des Standes der Technik war es nun Aufgabe der vorliegenden Erfindung, ein Verfahren zur Herstellung von Ethylenglycoldimethacrylat zur Verfügung zu stellen, bei dem das Produkt sehr kostengünstig erhalten werden kann. Darüber hinaus sollte das erhaltene Ethylenglycoldimethacrylat nur sehr geringe Mengen an Nebenprodukten und Katalysatorresten enthalten.

Eine weitere Aufgabe der Erfindung bestand darin, ein Verfahren zu schaffen, bei dem Ethylenglycoldimethacrylat sehr selektiv erhalten werden kann.

Darüber hinaus war es Aufgabe der vorliegenden Erfindung, Verfahren zur Herstellung von Ethylenglycoldimethacrylat zur Verfügung zu stellen, die einfach und kostengünstig durchgeführt werden können. Hierbei sollte das Produkt möglichst in hohen Ausbeuten und, insgesamt gesehen, unter geringem Energieverbrauch erhalten werden.

Gelöst werden diese sowie weitere nicht explizit genannte Aufgaben, die jedoch aus den hierin einleitend diskutierten Zusammenhängen ohne Weiteres ableitbar oder erschließbar sind, durch Verfahren mit allen Merkmalen des Patentanspruchs 1. Zweckmäßige Abwandlungen der erfindungsgemäßen Verfahren werden in den auf Anspruch 1 rückbezogenen abhängigen Ansprüchen unter Schutz gestellt.

Gegenstand der vorliegenden Erfindung ist dementsprechend ein Verfahren zur Herstellung von Ethylenglycoldimethacrylat, umfassend die Umesterung von Ethylenglycol mit einem Ester der Methacrylsäure in Gegenwart von Katalysatoren, wobei als Katalysator eine Kombination eingesetzt wird, die Lithiumamid und Lithiumchlorid umfasst.

Hierdurch gelingt es auf nicht vorhersehbare Weise ein Verfahren zur Herstellung von Ethylenglycoldimethacrylat zur Verfügung zu stellen, bei dem das Produkt sehr kostengünstig erhalten wird. Überraschend enthält das erhaltene Produkt nur sehr geringe Mengen an Nebenprodukten und Katalysatorresten.

Des Weiteren ermöglicht das erfindungsgemäße Verfahren eine besonders selektive Herstellung von Ethylenglycoldimethacrylat.

Weiterhin kann das erfindungsgemäße Verfahren einfach und kostengünstig durchgeführt werden, wobei das Produkt in hohen Ausbeuten und, insgesamt gesehen, unter geringem Energieverbrauch erhalten werden kann.

Erfindungsgemäß wird Ethylenglycoldimethacrylat (2-Methylpropensäure-1,2-ethandiyldiester) hergestellt, wobei Ethylenglycoldimethacrylat die CAS-Nummer 97-90-5 besitzt.

Zur Herstellung von Ethylenglycoldimethacrylat wird erfindungsgemäß Ethylenglycol (Ethan-1,2-diol) eingesetzt. Diese Verbindung ist kommerziell von BASF, Dow oder Shell erhältlich und besitzt die CAS-Nummer 107-21-1.

Gemäß der vorliegenden Erfindung wird Ethylenglycol mit einem Ester der Methacrylsäure umgesetzt. Besonders geeignete Methacrylate werden insbesondere von Alkoholen mit 1 bis 4 Kohlenstoffatomen gebildet. Zu diesen gehören insbesondere Methanol, Ethanol, n-Propanol, iso-Propanol, n-Butanol und tert.-Butanol. Besonders bevorzugt wird insbesondere Ethylmethacrylat oder Methylmethacrylat eingesetzt, wobei Methylmethacrylat ganz besonders bevorzugt ist.

Das Gewichtsverhältnis von Ethylenglycol zum Ester der Methacrylsäure liegt vorzugsweise im Bereich von 1:2 bis 1:20, besonders bevorzugt 1:5 bis 1:15 und ganz besonders bevorzugt im Bereich von 1:6 bis 1:10.

Erfindungsgemäß wird zur Katalyse der vorliegenden Umesterung eine Kombination eingesetzt, die Lithiumamid (LiNH₂) und Lithiumchlorid (LiCl) umfasst. Lithiumamid besitzt die CAS-Nummer 7782-89-0, Lithiumchlorid die CAS-Nummer 7447-41-8.

Das Gewichtverhältnis von Lithiumamid zu Lithiumchlorid kann je nach Reaktionsbedingungen in einem weiten Bereich liegen. Zweckmäßig kann dieses Verhältnis beispielsweise im Bereich von 20:1 bis 1:20, besonders bevorzugt im Bereich von 5:1 bis 1:1 liegen.

Die Menge an eingesetztem Katalysator kann in einem weiten Bereich liegen. Von besonderem Interesse sind jedoch Verfahren, bei denen der Anteil an Katalysator, bezogen auf das Gewicht des eingesetzten Ethylenglycols, im Bereich von 0,05 bis 8 Gew.-%, vorzugsweise im Bereich von 0,01 bis 5 Gew.-% und besonders bevorzugt im Bereich von 0,1 bis 1 Gew.-% liegt.

Die eingesetzte Gesamtmenge des Katalysators kann zu Beginn der Reaktion in die Reaktionsmischung gegeben werden. Gemäß einer besonders zweckmäßigen Abwandlung kann ein Teil des Katalysators, vorzugsweise ein Teil des Lithiumamids im Verlauf der Reaktion der Reaktionsmischung beigefügt werden. Vorzugsweise kann nach einem Umsatz im Bereich von 20 bis 80%, besonders bevorzugt im Bereich von 30% bis 60%, bezogen auf das Gewicht des eingesetzten Ethylenglycols, weiterer Katalysator in die Reaktionsmischung hinzugefügt werden. Von besonderem Interesse sind insbesondere Verfahren, bei denen mindestens 10 Gew.-%, besonders bevorzugt mindestens 20 Gew.-% des Lithiumamids während der Umsetzung in die Reaktionsmischung hinzu gegeben werden.

Die Umsetzung kann bei Über- oder Unterdruck erfolgen. Gemäß einer besonders zweckmäßigen Abwandlung der vorliegenden Erfindung kann die Umesterung bei einem Druck im Bereich von 200 bis 2000 mbar, besonders bevorzugt im Bereich von 500 bis 1300 mbar durchgeführt werden.

Die Reaktionstemperatur kann, insbesondere in Abhängigkeit des Druckes, ebenfalls in einem weiten Bereich liegen. Gemäß einer bevorzugten Ausführungsform der vorliegenden Erfindung erfolgt die Umsetzung vorzugsweise bei einer Temperatur im Bereich von 60°C bis 150°C, besonders bevorzugt im Bereich von 70°C bis 140°C und ganz besonders bevorzugt 90 bis 130 °C.

Überraschend können besondere Vorteile erzielt werden, falls die Temperatur, bei der die Umsetzung erfolgt, im Verlauf der Umsetzung erhöht wird. Gemäß dieser bevorzugten Abwandlung des erfindungsgemäßen Verfahrens kann die Temperatur zu Beginn der Umsetzung, insbesondere bis zu einem Umsatz von 80%, bevorzugt bis zu einem Umsatz von 70%, bezogen auf das Gewicht des eingesetzten Ethylenglycols, vorzugsweise im Bereich von 90°C bis 110°C und gegen Ende der Umsetzung, insbesondere nach einem Umsatz von 80%, bevorzugt nach einem Umsatz von 90%, bezogen auf das Gewicht des eingesetzten Ethylenglycols, im Bereich von 115 °C bis 130 °C liegen.

Die Umesterung kann sowohl kontinuierlich als auch chargenweise durchgeführt werden. Das erfindungsgemäße Verfahren kann in Substanz, d.h. ohne Verwendung eines weiteren Lösungsmittels durchgeführt werden. Falls gewünscht kann auch ein inertes Lösungsmittel eingesetzt werden. Hierzu gehören unter anderem Benzol, Toluol, n-Hexan, Cyclohexan und Methylisobutylketon (MIBK) und Methylethylketon (MEK).

Bei einer besonders zweckmäßigen Varianten der erfindungsgemäßen Umesterung werden sämtliche Komponenten, wie beispielsweise das Ethylenglycol, der Methacrylsäureester sowie der Katalysator, gemischt, wonach diese Reaktionsmischung bis zum Sieden erwärmt wird. Anschließend kann der frei werdende Alkohol, beispielsweise Methanol oder Ethanol, destillativ, gegebenenfalls azeotrop mit Methylmethacrylat oder Ethylmethacrylat, aus der Reaktionsmischung entfernt werden.

Die Reaktionszeiten sind unter anderem von den gewählten Parametern, wie zum Beispiel Druck und Temperatur, abhängig. Sie liegen aber im Allgemeinen im Bereich von 1 bis 24 Stunden, bevorzugt von 5 bis 20 Stunden und ganz besonders bevorzugt 6 bis 12 Stunden. Bei kontinuierlichen Verfahren liegen die Verweilzeiten im Allgemeinen im Bereich von 0,5 bis 24 Stunden, bevorzugt von 1 bis 12 Stunden und ganz besonders bevorzugt 2 bis 3 Stunden. Weitere Hinweise in Bezug auf die Reaktionszeiten kann der Fachmann den beigefügten Beispielen entnehmen.

Vorzugsweise kann die Reaktion unter Rühren stattfinden, wobei die Rührgeschwindigkeit besonders bevorzugt im Bereich von 50 bis 2000 Upm, ganz besonders bevorzugt im Bereich von 100 bis 500 Upm liegen kann.

Der pH-Wert kann in einem weiten Bereich liegen. Zweckmäßig kann die Umsetzung bei einem pH-Wert im Bereich von 8 bis 14, vorzugsweise 9 bis 13 durchgeführt werden.

Um eine unerwünschte Polymerisation der Methacrylate zu verhindern, können bei der Umsetzung Polymerisationsinhibitoren eingesetzt werden. Diese Verbindungen, wie beispielsweise Hydrochinone, Hydrochinonether, wie Hydrochinonmonomethylether oder Di-tert-butylbrenzcatechin, Phenothiazin, N,N'-(Diphenyl)-p-phenylendiamin, 4-Hydroxy-2,2,6,6-tetramethylpiperidin-1-oxyl, p-Phenylendiamin, Methylenblau oder sterisch gehinderte Phenole, sind in der Fachwelt weithin bekannt. Diese Verbindungen können einzeln oder in Form von Mischungen eingesetzt werden und sind im Allgemeinen kommerziell erhältlich. Die Wirkung der Stabilisatoren besteht meist darin, dass sie als Radikalfänger für die bei der Polymerisation auftretenden freien Radikale wirken. Für weitere Details wird auf die gängige Fachliteratur, insbesondere auf das Römpp-Lexikon Chemie; Herausgeber: J. Falbe, M. Regitz; Stuttgart, New York; 10. Auflage (1996); Stichwort "Antioxidantien" und die an dieser Stelle zitierten Literaturstellen verwiesen.

Vorzugsweise werden insbesondere Amine als Polymerisationsinhibitor eingesetzt. Besonders überraschende Vorteile können bei Verwendung von N,N'-(Diphenyl)-p-phenylendiamin erzielt werden. Bezogen auf das Gewicht der gesamten Reationsmischung kann der Anteil der Inhibitoren einzeln oder als Mischung im Allgemeinen 0,01 - 0,5 % (wt/wt) betragen.

Diese Polymerisationsinhibitoren können vor oder zu Beginn der Reaktion in die Reaktionsmischung gegeben werden. Darüber hinaus können auch Teile der beigefügten Polymerisationsinhibitoren während der Umesterung hinzu gegeben werden. Von besonderem Interesse sind hierbei insbesondere Verfahren bei denen ein Teil des Polymerisationsinhibitors über den Kolonnenrücklauf zugefügt wird. Besonders zweckmäßig sind unter anderem Mischungen, die Methylmethacrylat, Hydrochinonmonomethylether und 4-Hydroxy-2,2,6,6-tetramethylpiperidin-1-oxyl enthalten. Durch diese Maßnahme kann insbesondere eine unerwünschte Polymerisation innerhalb der Destillationskolonne vermieden werden.

Zur Inhibierung kann des Weiteren Sauerstoff verwendet werden. Hierbei kann dieser zum Beispiel in Form von Luft eingesetzt werden, wobei die Mengen vorteilhaft so dosiert werden, dass der Gehalt in der Gasphase über dem Reaktionsgemisch unterhalb der Explosionsgrenze bleibt. Besonders bevorzugt sind hierbei Luftmengen im Bereich von 0,05 bis 0,5 l pro Stunde und Mol Ethylenglycol. Bei Chargenverfahren kann sich diese Menge auf die ursprünglich eingesetzte Menge an Ethylenglycol beziehen. Bei kontinuierlichen Verfahren kann sich diese Menge auf die zugeführte Menge an Ethylenglycol beziehen. Ebenso können Inertgas-Sauerstoff-Gemische, z.B. Stickstoff-Sauerstoff- oder Argon-Sauerstoff-Gemische eingesetzt werden.

Gemäß einer besonderen Ausgestaltung der vorliegenden Erfindung kann zur Inhibierung eine Kombination von Sauerstoff mit mindestens einem Amin, vorzugsweise N,N'-(Diphenyl)-p-phenylendiamin, eingesetzt werden.

Entsprechend einer zweckmäßigen Ausführungsform der vorliegenden Erfindung kann der aus dem eingesetzten Methacrylat freigesetzte Alkohol, beispielsweise Methanol und/oder Ethanol, durch Destillation abgetrennt werden. Hierbei kann vorteilhaft beispielsweise eine Mischung abgetrennt werden, die Methylmethacrylat und Methanol enthält. Überraschend kann mit Vorteil ein Teil der abgetrennten Mischung in den folgenden Ansatz zurückgeführt werden. Gemäß dieser Abwandlung kann der zurückführbare Anteil der abgetrennten Mischung zu Ende der Reaktion, insbesondere nach einem Umsatz von 80%, bevorzugt nach einem Umsatz von 90% des eingesetzten Ethylenglycols, erhalten werden. Beispielsweise kann der Anteil der zurückgeführten Mischung zu Beginn des folgenden Ansatzes im Bereich von 10 bis 50%, bezogen auf die Gesamteinwaage an umzuesterndem Methacrylsäureester liegen.

Von besonderem Interesse sind unter anderem Chargenverfahren, bei denen während der Umesterung Methylmethacrylat zugegeben wird. Diese Ausführungsform ist beispielsweise von Vorteil, falls Methylmethacrylat zusammen mit Methanol aus der Reaktionsmischung entfernt wird. Vorzugsweise kann das Gewichtsverhältnis der Menge an während der Umesterung zugegebenem Methylmethacrylat zur Menge an abgetrenntem Methanol-Methylmethacrylat-Gemisch im Bereich von 2:1 bis 1:2 liegen.

Bei Chargenverfahren kann gegen Ende der Reaktion überschüssiges Edukt, insbesondere der nicht umgesetzte Ester der Methacrylsäure durch Destillation abgetrennt werden. Auch dieser kann im nächsten Batch ohne weitere Reinigung wieder eingesetzt werden.

Das zu Beginn der Umsetzung erhaltene methanol- oder ethanolreiche Destillat kann ebenfalls recycliert werden, beispielsweise durch Einarbeitung in eine im Verbund betriebene Anlage zur Herstellung des umzuesternden Methacrylatesters.

Eine geeignete Anlage zur Durchführung der vorliegenden Umesterung kann beispielsweise einen Rührkesselreaktor mit Rührwerk, Dampfheizung, Destillationskolonne und Kondensator umfassen. Derartige Anlagen sind an sich bekannt und beispielsweise in Ullmanns Encyclopedia of Industrial Chemistry (6. Auflage), Verlag Wiley-VCH, Weinheim 2003, Band 10, Seite 647, beschrieben. Die Größe der Anlage ist von der herzustellenden Menge an Ethylenglycoldimethacrylat abhängig, wobei das vorliegende Verfahren sowohl im Labormaßstab als auch in großtechnischem Maßstab durchgeführt werden kann. Gemäß einem besonderen Aspekt kann der Rührkesselreaktor dementsprechend ein Kesselvolumen im Bereich von 1m³ bis 30 m³, bevorzugt 3 m³ bis 20 m³ aufweisen. Das Rührwerk des Reaktorkessels kann insbesondere in Form eines Ankerrührers, Impellers, Schaufel-oder Inter-MIG-rührers ausgestaltet sein.

Die Aufgabe der Destillationskolonne ist es, sicherzustellen, daß ein methanol- bzw. ethanolreiches Azeotrop abgeführt wird um die Verluste an zwangsläufig mit ausgetragenem Eduktester zu minimieren. Die Destillationskolonne kann ein, zwei oder mehr Trennstufen besitzen. Als Anzahl der Trennstufen wird die Anzahl der Böden bei einer Bodenkolonne oder die Anzahl der theoretischen Trennstufen im Fall einer Packungskolonne oder eine Kolonne mit Füllkörpern bezeichnet. Beispiele für eine mehrstufige Destillationskolonne mit Böden beinhalten solche wie Glockenböden, Siebböden, Tunnelböden, Ventilböden, Schlitzböden, Sieb-Schlitzböden, Sieb-Glockenböden, Düsenböden, Zentrifugalböden, für eine mehrstufige Destillationskolonne mit Füllkörpern solche wie Raschig-Ringe, Lessing-Ringe, Pall-Ringe, Berl-Sättel, Intalox Sättel und für eine mehrstufige Destillationskolonne mit Packungen wie solche vom Typ Mellapak (Sulzer), Rombopak (Kühni), Montz-Pak (Montz). Durch die umsatzabhängige Anpassung des Rücklaufverhältnis gelingt es beispielsweise, bei Verwendung von Methylmethacrylat über weite Bereiche des Umsatzes einen Methanolanteil im Destillat einzustellen, der oberhalb von 60% liegt.

Zu den geeignete Kondensatoren, die in der Anlage zur Durchführung der vorliegenden Umesterung enthalten sein kann, gehören unter anderem Platten-und Rohrbündelwärmetauscher.

Nach Beendigung der Umsetzung genügt das erhaltene Ethylenglycoldimethacrylat bereits vielfach den zuvor dargelegten hohen Anforderungen, so dass eine weitere Aufreinigung vielfach nicht notwendig ist. Zur weiteren Qualitätssteigerung und insbesondere der Katalysatorabtrennung kann die erhaltene Mischung durch bekannte Verfahren aufgereinigt werden.

Gemäß einer Ausgestaltung des erfindungsgemäßen Verfahrens kann die erhaltene Produktmischung durch Filtrationsverfahren aufgereinigt werden. Diese Verfahren sind aus dem Stand der Technik bekannt (W. Gösele, Chr. Alt in Ullmann's Encyclopedia of Industrial Chemistry, (6. Auflage), Verlag Wiley-VCH, Weinheim 2003 , Band 13, Seiten731 und 746), wobei übliche Filtrationshilfsmittel, wie beispielsweise Aluminiumsilicat (Perlit) eingesetzt werden können. Beispielsweise können unter anderem kontinuierlich betreibbare Filter für eine Anschwemmfiltration oder Kerzenfilter verwendet werden.

Eine weitere Verbesserung der Qualität des Produkts kann beispielsweise durch eine Destillation des erhaltenen Filtrats erzielt werden. Wegen der Polymerisationsneigung des Monomeren sind Destillationsverfahren angeraten, bei denen die thermische Belastung des zu destillierenden Stoffes minimiert ist. Gut geeignet sind Vorrichtungen, bei denen das Monomer aus einer dünnen Schicht heraus kontinuierlich verdampft wird, wie Fallfilmverdampfer und Verdampfer mit einem rotierenden Wischersystem. Auch Kurzwegverdampfer können eingesetzt werden. Solche Vorrichtungen sind bekannt (Ullmanns Encyclopedia of Industrial Chemistry (6. Auflage), Verlag Wiley-VCH, Weinheim 2003, Band 36, Seite 505). So kann zum Beispiel ein kontinuierlicher Verdampfer mit rotierendem Wischersystem und aufgesetzter Kolonne eingesetzt werden. Die Destillation kann beispielsweise bei Druck im Bereich von 1 bis 40 mbar und einer Verdampfertemperatur von 120°C bis 150°C durchgeführt werden.

Nachfolgend soll die vorliegende Erfindung anhand von Beispielen und Vergleichsbeispielen erläutert werden, ohne dass hierdurch eine Beschränkung erfolgen soll.

### Beispiel 1:

In einem Rührkesselreaktor mit Rührwerk, Dampfheizung, Destillationskolonne und Kondensator werden 444 kg Ethylenglycol, 3018 kg Methacrylsäuremethylester (MMA), 0,167 kg N,N'-(Diphenyl)-p-phenylendiamin als Inhibitor und als Katalysator ein Gemisch aus 0,5 kg Lithiumamid, sowie 0,25 kg Lithiumchlorid vereint und unter Einleiten von Luft gerührt. Zur Kolonnenstabilisierung werden im Laufe der Reaktion insgesamt 151 kg MMA, die 0,24 kg Hydrochinonmonomethylether und 0,016 kg 4-Hydroxy-2,2,6,6-tetramethylpiperidin-1-oxyl gelöst enthalten in den Kolonnenrücklauf dosiert. Man erhitzt auf 97°C Sumpftemperatur, wobei die Kolonne zunächst unter vollständigem Rücklauf betrieben wird (ca. 15 min). Sobald die Temperatur am Kolonnenkopf unter 70°C fällt, zieht man unter einem Rücklaufverhältnis von 2:1 das Methanol-MMA-Gemisch ab. Dabei ergänzt man den MMA-Vorrat im Reaktor durch Zudosieren von gleichen Teilen MMA pro Teil abgezogenem Methanol-MMA-Gemisch. Innerhalb von 5 h werden so insgesamt 1320 kg MMA eingebracht. Nach dem Abzug von 450 l Methanol-MMA-Gemisch gibt man erneut 0,5 kg Lithiumamid hinzu. Innerhalb von 8 h wird das Rücklaufverhältnis bis auf 1,1:1 der abnehmenden Methanolentwicklung angepasst. Bei einer Sumpftemperatur von 130 °C ist die Reaktion beendet und man zieht überschüssiges MMA im Vakuum ab, wobei man den Druck allmählich bis auf 100 mbar reduziert. Wenn kein MMA mehr abdestilliert, wird das Vakuum aufgehoben. Der Kesselinhalt, bestehend aus dem katalysatorhaltigen Ethylenglycoldimethacrylat wird mit 10 kg Aluminiumsilicat (Perlit) als Filterhilfsmittel versetzt und per Anschwemmfiltration oder unter Zuhilfenahme eines Kerzenfilters vom Katalysator befreit.
Man erhält 1415 kg Rohester mit folgender Zusammensetzung (gaschromatographisch ermittelt):
Ethylenglycoldimethacrylat: 90 %
Ethylenglycolmonomethacrylat: 2,1 %
MMA: 0,9 %
(Methacryloyloxyethyl)-(3-methoxy)-isobutyrat : 0,25%
(Methacryloyloxyethyl)-(3-(methacryloyloxyethyl))-isobutyrat 4,2 %

Der Rohester wird in einen kontinuierlichen Verdampfer (Fläche 3,5 m²) mit rotierendem Wischersystem und aufgesetzter Kolonne, die ohne Rücklauf betrieben wird bei 5 mbar Druck und 135°C Verdampfertemperatur mit einer Rate von 300 kg /h eingespeist. Aus dem Austrag (280 kg /h) erhält man insgesamt 1320 kg Ethylenglycoldimethacrylat.
Zusammensetzung (gaschromatographisch ermittelt):
Ethylenglycoldimethacrylat: 98,4 %
Ethylenglycolmonomethacrylat: 0,86 %
MMA: 0,4 %
(Methacryloyloxyethyl)-(3-methoxy)-isobutyrat : 0,18%
(Methacryloyloxyethyl)-(3-(methacryloyloxyethyl))-isobutyrat 0,14 %

### Beispiel 2:

In einem Rührkesselreaktor mit Rührwerk, Dampfheizung, Destillationskolonne und Kondensator werden 336 kg Ethylenglycol, 3112 kg Methacrylsäuremethylester (MMA, bestehend aus 1600 kg frischem MMA und 1512 kg MMA aus der Vakuumphase des Beispiels 1), 0,125 kg N,N'-(Diphenyl)-p-phenylendiamin als Inhibitor und als Katalysator ein Gemisch aus 0,5 kg Lithiumamid, sowie 0,25 kg Lithiumchlorid vereint und unter Einleiten von Luft gerührt. Zur Kolonnenstabilisierung werden im Laufe der Reaktion insgesamt 151 kg MMA, die 0,24 kg Hydrochinonmonomethylether und 0,016 kg 4-Hydroxy-2,2,6,6-tetramethylpiperidin-1-oxyl gelöst enthalten in den Kolonnenrücklauf dosiert. Man erhitzt auf 93°C Sumpftemperatur, wobei die Kolonne zunächst unter vollständigem Rücklauf betrieben wird (ca. 15 min). Sobald die Temperatur am Kolonnenkopf unter 70°C fällt, zieht man unter einem Rücklaufverhältnis von 3 :1 das Methanol-MMA-Gemisch ab. Nach dem Abzug von 250 l Methanol-MMA-Gemisch gibt man erneut 0,5 kg Lithiumamid hinzu. Nach 8h erreicht man eine Sumpftemperatur von 130°C und die Reaktion ist beendet. Man zieht überschüssiges MMA im Vakuum ab, wobei man den Druck allmählich bis auf 100 mbar reduziert. Wenn kein MMA mehr abdestilliert, wird das Vakuum aufgehoben. Der Kesselinhalt, bestehend aus dem katalysatorhaltigen Ethylenglycoldimethacrylat wird mit 5 kg Aluminiumsilicat (Perlit) als Filterhilfsmittel versetzt und per Anschwemmfiltration oder unter Zuhilfenahme eines Kerzenfilters vom Katalysator befreit. Man erhält 1050 kg Rohester mit folgender Zusammensetzung (gaschromatographisch ermittelt):
Ethylenglycoldimethacrylat: 96,8 %
Ethylenglycolmonomethacrylat: 0,31 %
MMA: 0,75%
(Methacryloyloxyethyl)-(3-methoxy)-isobutyrat : 0,035%
(Methacryloyloxyethyl)-(3-(methacryloyloxyethyl))-isobutyrat 0,5 %

Der Rohester wird in einen kontinuierlichen Verdampfer (Fläche 3,5 m2) mit rotierendem Wischersystem und aufgesetzter Kolonne, die ohne Rücklauf betrieben wird bei 5 mbar Druck und 135°C Verdampfertemperatur mit einer Rate von 300 kg /h eingespeist. Aus dem Austrag (280 kg /h) erhält man insgesamt 980 kg Ethylenglycoldimethacrylat.
Zusammensetzung (gaschromatographisch ermittelt):
Ethylenglycoldimethacrylat: 98,8 %
Ethylenglycolmonomethacrylat: 0,68 %
MMA: 0,32 %
(Methacryloyloxyethyl)-(3-methoxy)-isobutyrat : 0,05%
(Methacryloyloxyethyl)-(3-(methacryloyloxyethyl))-isobutyrat 0,037 %

### Beispiel 3:

In einem Rührkesselreaktor mit Rührwerk, Dampfheizung, Destillationskolonne und Kondensator werden 1057 kg Ethylenglycol, 4290 kg Methacrylsäuremethylester (MMA), 0,350 kg N,N'-(Diphenyl)-p-phenylendiamin und 0,16 kg Hydrochinonmonomethylether sowie 0,65 kg einer 5%igen Lösung von 4-Hydroxy-2,2,6,6-tetramethylpiperidin-1-oxyl in MMA als Inhibitoren und als Katalysator ein Gemisch aus 1,45 kg Lithiumamid, sowie 0,7 kg Lithiumchlorid vereint und unter Einleiten von Luft gerührt. Zur Kolonnenstabilisierung werden im Laufe der Reaktion insgesamt 151 kg MMA, die 0,36 kg Hydrochinonmonomethylether und 0,019 kg 4-Hydroxy-2,2,6,6-tetramethylpiperidin-1-oxyl gelöst enthalten in den Kolonnenrücklauf dosiert. Man erhitzt auf 97°C Sumpftemperatur, wobei die Kolonne zunächst unter vollständigem Rücklauf betrieben wird (ca. 15 min). Sobald die Temperatur am Kolonnenkopf unter 70°C fällt, zieht man unter einem Rücklaufverhältnis von 2:1 das Methanol-MMA-Gemisch ab.

Dabei ergänzt man den MMA-Vorrat im Reaktor durch Zudosieren von gleichen Teilen MMA pro Teil abgezogenem Methanol-MMA-Gemisch. Innerhalb von 10 h werden so insgesamt 3442 kg MMA eingebracht. Nach dem Abzug von 800 l Methanol-MMA-Gemisch gibt man erneut 1 kg Lithiumamid hinzu, nach 2400 l Gemischabzug nochmals 0,5 kg. Bei einer Sumpftemperatur von 130 °C ist die Reaktion beendet und man zieht überschüssiges MMA im Vakuum ab, wobei man den Druck allmählich bis auf 100 mbar reduziert. Wenn kein MMA mehr abdestilliert, wird das Vakuum aufgehoben. Der Kesselinhalt, bestehend aus dem katalysatorhaltigen Ethylenglycoldimethacrylat wird mit 10 kg Aluminiumsilicat (Perlit) als Filterhilfsmittel versetzt und per Anschwemmfiltration oder unter Zuhilfenahme eines Kerzenfilters vom Katalysator befreit. Man erhält 3370 kg Rohester mit folgender Zusammensetzung (gaschromatographisch ermittelt):
Ethylenglycoldimethacrylat: 89 %
Ethylenglycolmonomethacrylat: 2,8 %
MMA: 1 %
(Methacryloyloxyethyl)-(3-methoxy)-isobutyrat : 0,28%
(Methacryloyloxyethyl)-(3-(methacryloyloxyethyl))-isobutyrat 5 %

Der Rohester wird in einen kontinuierlichen Verdampfer (Fläche 3,5 m2) mit rotierendem Wischersystem und aufgesetzter Kolonne, die ohne Rücklauf betrieben wird bei 5 mbar Druck und 135°C Verdampfertemperatur mit einer Rate von 400 kg /h eingespeist. Aus dem Austrag (380 kg /h) erhält man insgesamt 3200 kg Ethylenglycoldimethacrylat.
Zusammensetzung (gaschromatographisch ermittelt):
Ethylenglycoldimethacrylat: 97,1 %
Ethylenglycolmonomethacrylat: 1,7 %
MMA: 0,27 %
(Methacryloyloxyethyl)-(3-methoxy)-isobutyrat : 0,38%
(Methacryloyloxyethyl)-(3-(methacryloyloxyethyl))-isobutyrat 0,28 %

### Vergleichsbeispiel 1 (Ersatz von Lithiumamid durch Kaliummethanolat):

In einem 21- Rundkolben mit Rührwerk, elektrischer Beheizung, Destillationskolonne und Kühler werden 280 g Ethylenglycol, 1126 g Methacrylsäuremethylester (MMA), 0,1 g Hydrochinonmonomethylether sowie 0,01 g 4-Hydroxy-2,2,6,6-tetramethylpiperidin-1-oxyl als Inhibitoren vereint und unter Einleiten von Luft gerührt. Man erhitzt zum Sieden und destilliert 77 g eines MMA-Wasser-Gemischs ab. Man kühlt auf 74 °C Sumpftemperatur ab und fügt 77 g reines MMA als Ergänzung hinzu sowie als Katalysator ein Gemisch aus 2,73 g Kaliummethanolat als 32 %ige Lösung in Methanol und 0,26 g Lithiumchlorid. Man erhitzt auf 87°C Sumpftemperatur. Sobald die Temperatur am Kolonnenkopf unter 70°C fällt, zieht man unter einem Rücklaufverhältnis von 5 :1 das Methanol-MMA-Gemisch ab. Dabei ergänzt man den MMA-Vorrat im Reaktor durch Zudosieren von gleichen Teilen MMA pro Teil abgezogenem Methanol-MMA-Gemisch. Innerhalb von 5 h werden so insgesamt 676 g MMA eingebracht. Nach 2h gibt man erneut 1,36 g Kaliummethanolat als 32 %ige Lösung in Methanol und 0,26 g Lithiumchlorid hinzu. Bei einer Sumpftemperatur von 120 °C ist die Reaktion beendet und man zieht überschüssiges MMA im Vakuum ab, wobei man den Druck allmählich bis auf 8 bis10 mbar reduziert. Wenn kein MMA mehr abdestilliert, wird das Vakuum aufgehoben. Der Kolbeninhalt, bestehend aus dem katalysatorhaltigen Ethylenglycoldimethacrylat wird mit einer Filterschicht der Firma Seitz, Typ T120 per Druckfiltration vom Katalysator befreit. Man erhält 731 g Rohester mit folgender Zusammensetzung (gaschromatographisch ermittelt):
Ethylenglycoldimethacrylat: 68,5 %
Ethylenglycolmonomethacrylat: 20,2 %
MMA: 0,47 %
(Methacryloyloxyethyl)-(3-methoxy)-isobutyrat : 0,76%
(Methacryloyloxyethyl)-(3-(methacryloyloxyethyl))-isobutyrat 4 %
3-((Methacryloyloxyethyl)-methylisobutyrat : 2 %

### Vergleichsbeispiel 2 (Lithiummethanolat an Stelle von Lithiumamid):

In einem 21- Rundkolben mit Rührwerk, elektrischer Beheizung, Destillationskolonne und Kühler werden 248 g Ethylenglycol, 1200 g Methacrylsäuremethylester (MMA), 0,09 g Hydrochinonmonomethylether sowie 0,008 g 4-Hydroxy-2,2,6,6-tetramethylpiperidin-1-oxyl als Inhibitoren vereint und unter Einleiten von Luft gerührt. Man erhitzt zum Sieden und destilliert 67 g eines MMA-Wasser-Gemischs ab. Man kühlt auf 85 °C Sumpftemperatur ab und fügt 67 g reines MMA als Ergänzung hinzu sowie als Katalysator 0,38 g Lithiummethanolat. Man erhitzt auf 87°C Sumpftemperatur. Sobald die Temperatur am Kolonnenkopf unter 70°C fällt, zieht man unter einem Rücklaufverhältnis von 5 :1 das Methanol-MMA-Gemisch ab. Dabei ergänzt man den MMA-Vorrat im Reaktor durch Zudosieren von gleichen Teilen MMA pro Teil abgezogenem Methanol-MMA-Gemisch. Innerhalb von 4 h werden so insgesamt 601 g MMA eingebracht. Nach 2,5 h gibt man erneut 0,38 g Lithiummethanolat hinzu. Bei einer Sumpftemperatur von 125 °C ist die Reaktion beendet und man zieht überschüssiges MMA im Vakuum ab, wobei man den Druck allmählich bis auf 8 bis 10 mbar reduziert. Wenn kein MMA mehr abdestilliert, wird das Vakuum aufgehoben. Der Kolbeninhalt, bestehend aus dem katalysatorhaltigen Ethylenglycoldimethacrylat wird mit 4 g Tonsil 312 FF (Montmorillonit der Fa. Südchemie) als Adsorbens und einer Filterschicht der Firma Seitz, Typ T120 per Druckfiltration vom Katalysator befreit. Man erhält 728 g Rohester mit folgender Zusammensetzung (gaschromatographisch ermittelt):
Ethylenglycoldimethacrylat: 83,7 %
Ethylenglycolmonomethacrylat: 3,2 %
MMA: 0,66 %
(Methacryloyloxyethyl)-(3-methoxy)-isobutyrat : 1,9%
(Methacryloyloxyethyl)-(3-(methacryloyloxyethyl))-isobutyrat 5,8 %
3-((Methacryloyloxyethyl)-methylisobutyrat : 2,7 %

### Vergleichsbeispiel 3 (Natriummethanolat an Stelle von Lithiumamid)

In einem 21- Rundkolben mit Rührwerk, elektrischer Beheizung, Destillationskolonne und Kühler werden 280 g Ethylenglycol, 826 g Methacrylsäuremethylester (MMA) sowie 300 g MMA aus dem Vakuumabzug des Beispiels 4 und 0,1 g Hydrochinonmonomethylether als Inhibitor vereint und unter Einleiten von Luft gerührt. Man erhitzt zum Sieden und destilliert 71 g eines MMA-Wasser-Gemischs ab. Man kühlt auf 73 °C Sumpftemperatur ab und fügt 71 g reines MMA als Ergänzung hinzu sowie als Katalysatoren 1,82 g Natriummethanolat in Form der 30%igen Lösung in Methanol und 0,85 g Lithiumchlorid. Man erhitzt auf 88°C Sumpftemperatur. Sobald die Temperatur am Kolonnenkopf unter 70°C fällt, zieht man unter einem Rücklaufverhältnis von 5 :1 das Methanol-MMA-Gemisch ab. Dabei ergänzt man den MMA-Vorrat im Reaktor durch Zudosieren von gleichen Teilen MMA pro Teil abgezogenem Methanol-MMA-Gemisch. Innerhalb von 3 h werden so insgesamt 450 g MMA eingebracht. Nach 1 h gibt man erneut 0,91g Natriummethanolat in Form der 30%igen Lösung in Methanol und 0,43 g Lithiumchlorid hinzu. Bei einer Sumpftemperatur von 128 °C ist die Reaktion beendet und man zieht überschüssiges MMA im Vakuum ab, wobei man den Druck allmählich bis auf 8 bis 10 mbar reduziert. Wenn kein MMA mehr abdestilliert, wird das Vakuum aufgehoben. Der Kolbeninhalt, bestehend aus dem katalysatorhaltigen Ethylenglycoldimethacrylat wird mit 4,5 g Tonsil 312 FF (Montmorillonit der Fa. Südchemie) als Adsorbens und einer Filterschicht der Firma Seitz, Typ T750 per Druckfiltration vom Katalysator befreit. Man erhält 828 g Rohester mit folgender Zusammensetzung (gaschromatographisch ermittelt):
Ethylenglycoldimethacrylat: 85,4 %
Ethylenglycolmonomethacrylat: 1,1%
MMA: 0,05 %
(Methacryloyloxyethyl)-(3-methoxy)-isobutyrat : 1,3%
(Methacryloyloxyethyl)-(3-(methacryloyloxyethyl))-isobutyrat 8 %
3-((Methacryloyloxyethyl)-methylisobutyrat : 2,4 %

### Vergleichsbeispiel 4 (Lithiumnitrat an Stelle von Lithiumchlorid)

In einem 2I- Rundkolben mit Rührwerk, elektrischer Beheizung, Destillationskolonne und Kühler werden 280 g Ethylenglycol und 1126 g Methacrylsäuremethylester (MMA) sowie 0,178 g Hydrochinonmonomethylether und 0,01 g 4-Hydroxy-2,2,6,6-tetramethylpiperidin-1-oxyl als Inhibitoren vereint und unter Einleiten von Luft gerührt. Man erhitzt zum Sieden und destilliert 81 g eines MMA-Wasser-Gemischs ab. Man kühlt auf 66 °C Sumpftemperatur ab und fügt 81 g reines MMA als Ergänzung hinzu sowie als Katalysatoren 3,07 g Natriummethanolat in Form der 30%igen Lösung in Methanol und 1,17 g Lithiumnitrat. Man erhitzt auf 87°C Sumpftemperatur. Sobald die Temperatur am Kolonnenkopf unter 70°C fällt, zieht man unter einem Rücklaufverhältnis von 5 :1 das Methanol-MMA-Gemisch ab. Dabei ergänzt man den MMA-Vorrat im Reaktor durch Zudosieren von gleichen Teilen MMA pro Teil abgezogenem Methanol-MMA-Gemisch. Innerhalb von 5 h werden so insgesamt 450 g MMA eingebracht. Nach 1,25 h gibt man erneut 1g Natriummethanolat in Form der 30%igen Lösung in Methanol und 0,39 g Lithiumnitrat hinzu. Bei einer Sumpftemperatur von 121 °C ist die Reaktion beendet und man zieht überschüssiges MMA im Vakuum ab, wobei man den Druck allmählich bis auf 5...6 mbar reduziert. Wenn kein MMA mehr abdestilliert, wird das Vakuum aufgehoben. Der Kolbeninhalt, bestehend aus dem katalysatorhaltigen Ethylenglycoldimethacrylat wird mit einer Filterschicht der Firma Seitz, Typ T 120 per Druckfiltration vom Katalysator befreit. Man erhält 706 g Rohester mit folgender Zusammensetzung (gaschromatographisch ermittelt):
Ethylenglycoldimethacrylat: 70 %
Ethylenglycolmonomethacrylat: 22,6%
MMA: 1 %
(Methacryloyloxyethyl)-(3-methoxy)-isobutyrat : 0,4%
(Methacryloyloxyethyl)-(3-(methacryloyloxyethyl))-isobutyrat 3,1%
3-((Methacryloyloxyethyl)-methylisobutyrat : 1,1 %

## Patentansprüche

1. Verfahren zur Herstellung von Ethylenglycoldimethacrylat, umfassend die Umesterung von Ethylenglycol mit einem Ester der Methacrylsäure in Gegenwart von Katalysatoren, **dadurch gekennzeichnet, dass** als Katalysator eine Kombination eingesetzt wird, die Lithiumamid (LiNH₂) und Lithiumchlorid (LiCl) umfasst.

2. Verfahren gemäß Anspruch 1, **dadurch gekennzeichnet, dass** ein Teil des Lithiumamids im Verlauf der Reaktion der Reaktionsmischung beigefügt wird.

3. Verfahren gemäß Anspruch 1 oder 2, **dadurch gekennzeichnet, dass** das Gewichtsverhältnis von Lithiumamid zu Lithiumchlorid im Bereich von 20:1 bis 1:20 liegt

4. Verfahren gemäß mindestens einem der vorhergehenden Ansprüche, **dadurch gekennzeichnet, dass** die Reaktionszeit im Bereich von 5 bis 20 Stunden liegt.

5. Verfahren gemäß mindestens einem der vorhergehenden Ansprüche, **dadurch gekennzeichnet, dass** Methylmethacrylat als Ester der Methacrylsäure eingesetzt wird.

6. Verfahren gemäß mindestens einem der vorhergehenden Ansprüche, **dadurch gekennzeichnet, dass** Ethylmethacrylat als Ester der Methacrylsäure eingesetzt wird.

7. Verfahren gemäß mindestens einem der vorhergehenden Ansprüche, **dadurch gekennzeichnet, dass** das Gewichtsverhältnis von Ethylenglycol zum Ester der Methacrylsäure im Bereich von 1:2 bis 1:20 liegt.

8. Verfahren gemäß mindestens einem der vorhergehenden Ansprüche, **dadurch gekennzeichnet, dass** die Umsetzung bei einem Druck im Bereich von 200 bis 2000 mbar erfolgt.

9. Verfahren gemäß mindestens einem der vorhergehenden Ansprüche, **dadurch gekennzeichnet, dass** die Temperatur zu Beginn der Umsetzung im Bereich von 90°C bis 110°C und gegen Ende der Umsetzung im Bereich von 115 °C bis 130 °C liegt.

10. Verfahren gemäß mindestens einem der vorhergehenden Ansprüche, **dadurch gekennzeichnet, dass** die Umsetzung in Gegenwart eines Polymerisationsinhibitors erfolgt.

11. Verfahren gemäß mindestens einem der vorhergehenden Ansprüche, **dadurch gekennzeichnet, dass** die Umsetzung unter Einleitung von Sauerstoff erfolgt.

12. Verfahren gemäß mindestens einem der vorhergehenden Ansprüche, **dadurch gekennzeichnet, dass** der aus dem eingesetzten Ester der Methacrylsäure freigesetzte Alkohol durch Destillation abgetrennt wird.

13. Verfahren gemäß Anspruch 12, **dadurch gekennzeichnet, dass** eine Mischung abgetrennt wird, die Methylmethacrylat und Methanol enthält.

14. Verfahren gemäß einem der Ansprüche 12 oder 13, **dadurch gekennzeichnet, dass** ein Teil der abgetrennten Mischung in den folgenden Ansatz zurückgeführt wird.

15. Verfahren gemäß Anspruch 14, **dadurch gekennzeichnet, dass** der zurückführbare Anteil der abgetrennten Mischung gegen Ende der Reaktion gewonnen wird.

16. Verfahren gemäß mindestens einem der vorhergehenden Ansprüche, **dadurch gekennzeichnet, dass** während der Umesterung Methylmethacrylat zugegeben wird.

17. Verfahren gemäß Anspruch 16, **dadurch gekennzeichnet, dass** das Gewichtsverhältnis der Menge an während der Umesterung zugegebenem Methylmethacrylat zur Menge an abgetrenntem Methanol-Methylmethacrylat-Gemisch im Bereich von 2:1 bis 1:2 liegt.

## Claims

1. Process for preparing ethylene glycol dimethacrylate, which comprises transesterification of ethylene glycol with an ester of methacrylic acid in the presence of catalysts, **characterized in that** a combination comprising lithium amide (LiNH₂) and lithium chloride (LiCl) is used as catalyst.

2. Process according to Claim 1, **characterized in that** part of the lithium amide is added to the reaction mixture during the course of the reaction.

3. Process according to Claim 1 or 2, **characterized in that** the weight ratio of lithium amide to lithium chloride is in the range from 20:1 to 1:20.

4. Process according to at least one of the preceding claims, **characterized in that** the reaction time is in the range from 5 to 20 hours.

5. Process according to at least one of the preceding claims, **characterized in that** methyl methacrylate is used as ester of methacrylic acid.

6. Process according to at least one of the preceding claims, **characterized in that** ethyl methacrylate is used as ester of methacrylic acid.

7. Process according to at least one of the preceding claims, **characterized in that** the weight ratio of ethylene glycol to the ester of methacrylic acid is in the range from 1:2 to 1:20.

8. Process according to at least one of the preceding claims, **characterized in that** the reaction is carried out at a pressure in the range from 200 to 2000 mbar.

9. Process according to at least one of the preceding claims, **characterized in that** the temperature at the beginning of the reaction is in the range from 90°C to 110°C and that towards the end of the reaction it is in the range from 115°C to 130°C.

10. Process according to at least one of the preceding claims, **characterized in that** the reaction is carried out in the presence of a polymerization inhibitor.

11. Process according to at least one of the preceding claims, **characterized in that** the reaction is carried out with introduction of oxygen.

12. Process according to at least one of the preceding claims, **characterized in that** the alcohol liberated from the ester of methacrylic acid used is separated off by distillation.

13. Process according to Claim 12, **characterized in that** a mixture containing methyl methacrylate and methanol is separated off.

14. Process according to one of Claims 12 or 13, **characterized in that** part of the mixture which has been separated off has been recirculated to the following batch.

15. Process according to Claim 14, **characterized in that** the proportion which can be recirculated of the mixture which has been separated off can be obtained at the end of the reaction.

16. Process according to at least one of the preceding claims, **characterized in that** methyl methacrylate is added during the transesterification.

17. Process according to Claim 16, **characterized in that** the weight ratio of the amount of methyl methacrylate added during the transesterification to the amount of methanol/methyl methacrylate mixture separated off is in the range from 2:1 to 1:2.

## Revendications

1. Procédé de fabrication de diméthacrylate d'éthylène glycol, comprenant la transestérification d'éthylène glycol avec un ester de l'acide méthacrylique en présence de catalyseurs, **caractérisé en ce qu'**une combinaison qui comprend de l'amide de lithium (LiNH₂) et du chlorure de lithium (LiCl) est utilisée en tant que catalyseur.

2. Procédé selon la revendication 1, **caractérisé en ce qu'**une partie de l'amide de lithium est ajoutée au mélange réactionnel au cours de la réaction.

3. Procédé selon la revendication 1 ou 2, **caractérisé en ce que** le rapport en poids entre l'amide de lithium et le chlorure de lithium se situe dans la plage allant de 20:1 à 1:20.

4. Procédé selon au moins l'une quelconque des revendications précédentes, **caractérisé en ce que** la durée de réaction se situe dans la plage allant de 5 à 20 heures.

5. Procédé selon au moins l'une quelconque des revendications précédentes, **caractérisé en ce que** le méthacrylate de méthyle est utilisé en tant qu'ester de l'acide méthacrylique.

6. Procédé selon au moins l'une quelconque des revendications précédentes, **caractérisé en ce que** le méthacrylate d'éthyle est utilisé en tant qu'ester de l'acide méthacrylique.

7. Procédé selon au moins l'une quelconque des revendications précédentes, **caractérisé en ce que** le rapport en poids entre l'éthylène glycol et l'ester de l'acide méthacrylique se situe dans la plage allant de 1:2 à 1:20.

8. Procédé selon au moins l'une quelconque des revendications précédentes, **caractérisé en ce que** la réaction a lieu à une pression dans la plage allant de 200 à 2 000 mbar.

9. Procédé selon au moins l'une quelconque des revendications précédentes, **caractérisé en ce que** la température au début de la réaction se situe dans la plage allant de 90 °C à 110 °C et vers la fin de la réaction dans la plage allant de 115 °C à 130 °C.

10. Procédé selon au moins l'une quelconque des revendications précédentes, **caractérisé en ce que** la réaction a lieu en présence d'un inhibiteur de polymérisation.

11. Procédé selon au moins l'une quelconque des revendications précédentes, **caractérisé en ce que** la réaction a lieu avec introduction d'oxygène.

12. Procédé selon au moins l'une quelconque des revendications précédentes, **caractérisé en ce que** l'alcool libéré à partir de l'ester de l'acide méthacrylique utilisé est séparé par distillation.

13. Procédé selon la revendication 12, **caractérisé en ce qu'**un mélange contenant du méthacrylate de méthyle et du méthanol est séparé.

14. Procédé selon l'une quelconque des revendications 12 ou 13, **caractérisé en ce qu'**une partie du mélange séparé est recyclée dans la préparation suivante.

15. Procédé selon la revendication 14, **caractérisé en ce que** la fraction recyclable du mélange séparé est obtenue vers la fin de la réaction.

16. Procédé selon au moins l'une quelconque des revendications précédentes, **caractérisé en ce que** du méthacrylate de méthyle est ajouté pendant la transestérification.

17. Procédé selon la revendication 16, **caractérisé en ce que** le rapport en poids entre la quantité de méthacrylate de méthyle ajouté pendant la transestérification et la quantité de mélange méthanol-méthacrylate de méthyle séparé se situe dans la plage allant de 2:1 à 1:2.
